# EUROPEAN PATENT APPLICATION

(11) **EP 3 329 863 A1**
(43) Date of publication of application: **06.06.2018**
(21) Application number: 17188425.7
(22) Date of filing: 29.08.2017
(51) Int. Cl.: A61B 17/115, A61B 17/072, A61B 17/326, A61B 34/20

(54) **CIRCUMCISION STAPLER**

(30) Priority: 05.12.2016 CN 201611104805
(71) Applicant: Smartpro (Europe) GmbH, 10787 Berlin (DE)
(72) Inventor: Zhang, Lei, Wenzhou, Zhejiang (CN); Wu, Zhe, Wenzhou, Zhejiang (CN)
(74) Representative: Seliger, Knut

(57) **Abstract**

The present invention relates to a prepuce cutting and suturing device. An internal positioning member and an external positioning member on a staple seat of the device are matched to position prepuce. An operator is capable of holding the staple seat with one hand and turns the prepuce with another hand while observing the position of frenulum, adjusts the frenulum to prevent the frenulum from entering the cutting area. The operator pushes an annular cutter to cut the prepuce, pushes a staple pusher to move suturing staples toward staple grooves. When stapled on the prepuce, the suturing staples penetrate through one side of each corresponding one of cushions. One side of each one of the cushions with the suturing staples is stably positioned, while the other side turns along with penis erection, reducing the pressure between the suturing staples and the skin. Non-symmetric cushions drive the suturing staples get closer to the incision. When the penis erects, the suturing staples more easily cut the cut edge and get out of the cut edge by the levering effect of the leverage force of the single-sided cushions away from the cut edge. After the cutting and suturing is completed, the operator observes the situations of the suturing staples at the cut under the condition that the external positioning member is not removed. When ensuring that all suturing staples are firmly stapled, the external positioning member is allowed to be removed. If it is found that the suturing staples are not firmly stapled, corrective suturing is carried out before the external positioning member is removed, avoiding bleeding during operation.

## Description

### BACKGROUND OF THE INVENTION

### 1. Technical Field

The present invention relates to a medical device, in particular to a circumcision stapler.

### 2. Description of Related Art

Redundant prepuce and phimosis are the most common reproductive problems for men. Along with continuous improvement on people's living standard and health consciousness, higher requirements are imposed on the productive health. Redundant prepuce easily hides dirts and causes balano-posthitis, affecting normal sexual life and increasing the probability that the sexual partner suffers from uterine cervicitis and cervical cancer. Circumcision is an effective method for treating redundant prepuce and phimosis. At present, redundant prepuce is usually treated by the conventional circumcision or using cutting and suturing devices. The conventional circumcision adopts manual cutting and suturing operation, tending to cause un-smooth prepuce cut edge and relatively severe bleeding during the surgery and costing a long operation time. Existing cutting and suturing devices feature in smooth cut, shorter operation time and convenient procedure, but still have some defects.

By the cutting and suturing technologies of the existing devices, key structures such as the frenulum of prepuce cannot be accurately controlled, thus easily resulting in damage to the device, failure to standardize the surgery, deviation of the frenulum of prepuce after the surgery, and over-short or over-long prepuce, which affects the appearance. Besides, according to the cutting and suturing technologies of the existing devices, the suturing staples are simple structures, and after the stapling work is completed, the suturing staples are bent and formed into a clamping ring to loop the prepuce tissue. However, penis does not like other body parts, it erects sometimes such that the prepuce tissue where the suturing staple is fastened is stressed. Along with the time, such flesh tissue lacks blood and suffers necrosis, and then the suturing staple is penetrated and cut into the prepuce tissue, causing tissue damage and post-operation cicatrization. Once the suturing staple is penetrated into the prepuce tissue and wrapped by the prepuce tissue, it is very difficult to remove the staple, and the appearance is affected by scars. Finally, patients get hurt. In prior art, there are suturing staples matched with symmetrical cushions. However, when the penis erects, the uniformly stressed two sides of the cushion press the skin, and the skin tissue is damaged and generates scars which affect the appearance, and causing pains to patients. Moreover, the symmetrical cushions brings inconvenience in subsequent removal of the suturing staple. According to the hemostatic technologies of the existing devices, the intervals between staples are too wide and it is easy to miss blood vessels during stapling, thus causing bleeding or the complications of hematoma during and after the surgery. On the other hand, the existing devices have the defect that the operators cannot sight the accurate position of the frenulum of prepuce directly during the surgery. How much inner or outer plates of the prepuce have to be removed cannot be calculated, accurate positioning and fixation cannot be achieved, resulting in high risk in cutting or injuring the frenulum of prepuce during the surgery. The prepuce cannot be reliably fixed during the surgery, resulting in that the inner and outer plates of the prepuce shift in relative positions during the procedure, and resulting in deviation of the frenulum of prepuce and axial rotation deformity of the penis after the operation. After the prepuce is cut, there will be more or less some problems, for example the erection of the penis is affected after the surgery, or the appearance is unsatisfied.

### BRIEF SUMMARY OF THE INVENTION

The objective of the present invention is to provide a prepuce surgery device to overcome defects in the prior art. The device can reduce bleeding during and after surgery, improve the comfort level at the sutured prepuce, reduce tension during erection, and facilitate staple removal, reduce post-operative cicatrization. The device can provide a direct view to help adjustment and positioning of the frenulum of prepuce, adjust the length of the inner and outer plates of the prepuce, and perform good limiting fixation. The device is beneficial to reduction of the operation risks, increase in operation safety and improvement on operation quality, more standardizes the surgery, and improves operative repeatability.

The present invention adopts the following technical solution. A circumcision stapler includes an annular cutter, a staple pusher, a staple cartridge, a staple seat and an external positioning member; wherein the staple seat includes a staple seat body, a balanus accommodating cavity, staple slot grooves and an internal positioning member; the staple seat body has an inner cavity and an outer wall; the inner cavity has one end which is inward recessed and extends to form the balanus accommodating cavity; the outer wall is provided with a ring of staple grooves; the internal positioning member is formed between the balanus accommodating cavity and the outer wall of the staple grooves; wherein the annular cutter is disposed on inner side of the staple pusher; the staple pusher, the staple cartridge and the staple seat are disposed in turn; the staple cartridge is provided with staple compartments; a cushion compartment is disposed on one side of each one of the staple compartments; the staple grooves are disposed corresponding to the staple compartments and the cushion compartments; the projection position of each one of suturing staples in the staple compartment is close to the inner side of each one of the cushion compartments; each one of the cushion compartments contains single-sided staple cushions; wherein during use, the balanus (that is, the glans of the penis) is placed in the balanus accommodating cavity and the prepuce is turned to the outer wall of the staple seat body and hooped by the external positioning member from the outside; the external positioning member fixes the prepuce at the position of the internal positioning member; the staple cartridge is aligned with the prepuce at the outer wall of the staple seat body; the annular cutter and the staple pusher are pushed toward the staple grooves; the annular cutter cuts the prepuce, and then the staple pusher pushes the suturing staples and a single-sided staple cushion in the staple cartridge to the staple grooves; wherein the suturing staples are fastened on the prepuce, and the suturing staples are penetrated in one side of each one of the single-sided staple cushions; one side of each one of the single-sided staple cushions with the suturing staples is stably positioned, and the other side of each one of the single-sided staple cushion can flip along with the erection of the penis.

As an improvement, the opposite faces of the internal positioning member and the external positioning member are respectively provided with a clamping groove and a clamping ridge which are matched with each other; when the internal positioning member and the external positioning member are matched to perform positioning, the clamping ridge is limited by the clamping groove and groove walls on two sides, and the prepuce is limited by a fold-like space formed by the clamping groove and the clamping ridge.

As an improvement, the external positioning member is annular; the external positioning member is a multi-segment or elastic one-segment positioning arc; the multi-segment positioning arc is connected in turn and is able to open and close around the connection; the two outer ends of the positioning arc are formed into openings; end portions of the positioning arc at the openings are respectively provided with an upper snap joint and a lower snap joint which are matched and snap-fitted; the snap-fitted multi-segment positioning arc is ring formed; clutch handles which are operated by people are disposed on the outer sides of the reverse faces of the upper snap joint and the lower snap joint, and when two clutch handles are operated by a reverse acting force, the upper snap joint and the lower snap joint are separated from each other.

As an improvement, when the external positioning member hoops the prepuce from the outside at the position of the internal positioning member, the clearance between the internal positioning member and the external positioning member enables the positioned prepuce to adjust in position.

As an improvement, the staple seat also includes a multi-ridge columnar connecting bridge which is connected to the other end of the staple seat opposite to the balanus accommodating cavity, the internal positioning member is disposed on the outer circumference of the opening end of the balanus accommodating cavity on the staple seat; and the staple seat is formed with at least one view window which runs through the balanus accommodating cavity and through which operator can observe the positions of the balanus and the prepuce.

As an improvement, a main bracket and a handle are also included; the main bracket is internally fixedly provided with the staple cartridge; the main bracket is internally movably provided with the annular cutter and the staple pusher; the handle is rotationally disposed on the main bracket and penetrated in the main bracket to match with the staple pusher in a linked way; the handle includes an outer rotating arm and inner pushing arms; the outer rotating arm and the inner pushing arms are rotationally disposed on the main bracket through a middle rotating shaft; the rotating shaft is provided with a torsional spring; the torsional spring is matched with the outer rotating arm and the main bracket; two inner pushing arms are provided, and the two inner pushing arms extend into the symmetrical pushing grooves on the staple pusher; the main bracket is internally provided with a reset spring; the reset spring is matched with a stopper disposed on the staple pusher; the stopper compresses the reset spring when the staple pusher moves forward; the reset spring drives the staple pusher to reset when the force applied to the staple pusher is removed; a projection is disposed at the bottom of each one of the pushing grooves, and when the inner pushing arms are pressed against the bottoms of the pushing grooves and rotationally pass through the projections, the staple pusher is sprung back by the force of the reset spring and gives the prompt that the staple is pushed in place.

As an improvement, the main bracket is also provided with a pressing member and a pressing spring; the pressing member and the pressing spring are matched and are installed on the main bracket; when the staple seat is installed in the main bracket, the staple seat is pressed against the pressing member to compress the pressing spring; the far end of the staple seat penetrates out of the main bracket and is detachably provided with a locking knob; when rotating the locking knob, a person determines the position of the pressing member via a locking mark window disposed on the main bracket; the main bracket is also provided with a rotary clamp, the outer rotating arm is provided with clamping points, and when the rotary clamp is pressed against the clamping points, the outer rotating arm is limited.

As an improvement, the staple grooves are arrayed in parallel in the circumferential direction; intervals between adjacent staple grooves are all smaller than or equal to 1.0mm, and are all greater than or equal to 0.4mm; and the staple compartment and the cushion compartment are disposed in a way of corresponding to the staple grooves.

As an improvement, each one of the staple compartments has limiting grooves on one two sides for positioning suturing staple feet; the projections of the limiting grooves are positioned at the inner side of each one of the cushion compartments; the limiting grooves in each one of the staple compartments limit the suturing staples; each one of the cushion compartments includes a staple loading cushion cavity side and a staple levering cushion cavity side; the staple loading cushion cavity side and the limiting grooves are correspondingly disposed in terms of the projection positions; the staple levering cushion cavity side is positioned on the outer side of the staple loading cushion cavity side; each one of the single-sided staple cushions includes a staple loading portion and a staple levering portion which are integrated; the staple loading cushion cavity side and the staple levering cushion cavity side respectively accommodate the staple loading portion and the staple levering portion; the staple loading portion is penetrated with the suturing staples; the staple levering portion is a turnable side; after the suturing staple is fastened on the prepuce, the staple levering portion faces the root of the penis; and when the penis erects, the staple levering portion is stressed to turn toward the cutting direction.

As an improvement, the staple compartments and the cushion compartments are disposed close to the inner wall of the staple cartridge, and form axial run-through openings on the inner wall of the staple cartridge.

The present invention has the following beneficial effects: the circumcision stapler is applicable to suturing staples with single-sided cushions; the staple cartridge is provided with staple compartment and cushion compartment for respectively accommodating the suturing staples and staple cushions, and the limiting grooves where the suturing staples in the staple compartment are positioned are reasonably formed on the inner side of the cushion compartment; when the suturing staple is pushed out of the staple compartment, the suturing staple can be sleeved with the staple loading cushion which is positioned on one side of the staple shim to form a single-sided cushion mode, and then the combined body penetrates through the prepuce tissue to be stapled and then is molded by the chamfers of the staple grooves on the staple plate to hold and staple the prepuce, so that the staple shim is positioned between the prepuce tissue and the suturing staple. The large area of the cushion can ensure good contact with the skin tissue, and the single-sided cushions reduce the pressure on the skin tissue applied by the original suturing staples and can prevent the suturing staples from cutting and penetrating into the tissue or being wrapped by the tissue. The staple compartments are matched with the staple plate to press the prepuce, while the single-sided staple cushions and the suturing staples press the outer side of the cutting edge, and the annular cutter cuts the prepuce, thus effectively reducing bleeding during and or after the surgery. The shapes of the staple pusher and the staple compartments are adapted to the single-sided staple cushions and the suturing staples to improve the stability of staple pushing and cushion pushing, while the straight-push stapling mode ensures stable pushing and stapling of a ring of single-sided staple cushions and suturing staples. After the suturing staples with single-sided cushions are fastened on the prepuce, the single-sided staple cushions can keep good contact with the penis skin, reduce the pressure between the suturing staples and the skin tissue when the penis erects, improving the comfort level of the patient. Meanwhile, the pressure caused by the erection of the penis is undertaken by one side of the cushions, and the erecting penis generates an outward thrust to the aother side of the staple cushions, so a leverage force for removing the suturing staples toward the cutting edge is generated; the asymmetric cushions make the staple frame portions of the suturing staples closer to the cutting edge, and then the suturing staples more easily cut the cuting edge and get out of the cuting edge by the poking effect of the leverage of the single-sided staple cushions, thus achieving the effect of quick staple removal, minimizing the cuting edge scars after the operation, making the cuting edge natural and good-looking after healing, and generally improving the shaping effect. On the other hand, the staple seat is a compound structure; the balanus accommodating cavity plays a good role of accommodating and protecting the balanus; after being wrapped by the prepuce, the internal positioning member has a certain extension limiting effect; after the external positioning member and the internal positioning member are matched and positioned, the operator can hold the staple seat with one hand and turn the prepuce with the other hand to completely expose the frenulum of prepuce and the inner plate structure, then directly view the frenulum position, locate and adjust the frenulum of prepuce to prevent the frenulum of prepuce from entering the cutting and suturing zone of the staple grooves, and can rotate the staple seat to fine tune the frenulum of prepuce to align with the middle line of the ventral side of the outer plate. After ensuring that the frenulum of prepuce is secure and well aligned and the inner and outer plates are adjusted in good conditions, the skins of the inner and outer plates of the prepuce are fixed and limited, the staple cartridge and the staple seat of the prepuce cutting and suturing device are assembled, and the handle is pressed to push the cutter and the staple to complete prepuce cutting and suturing. The staple grooves of the staple seat are used for bending the staple feet and molding the suturing staple. After the cutting and suturing is completed, remove the staple cartridge. Under the condition that the external positioning member is not removed, observe the situation of the suturing staple at the cutting edge. At this time, the external positioning member plays a certain role of pressing the prepuce to stop bleeding. After ensuring that the all suturing staples are firmly fastened, remove the external positioning member. If it is found that the suturing staples are not firmly stapled, the corrective suturing is carried out before external positioning member is removed, avoiding the risk of bleeding during surgery. By the above technical improvements, the surgery is safer and more accurate, bleeding complications are reduced during the operation, the operation quality and efficiency are enhanced, and the operation satisfaction degree of the patients is comprehensively improved.

### BRIEF DESCRIPTION OF THE SEVERAL VIEWS OF THE DRAWINGS

Fig.1 is a three-dimensional exploded view of a prepuce cutting and suturing device of the present invention;
Fig.2 is a three-dimensional structural view of a seat staple of the present invention;
Fig.3 is a sectional view of the seat staple of the present invention;
Fig.4 is a sectional structural view of an external positioning member of the present invention;
Fig.5 is a sectional view of the circumcision stapler of the present invention;
Fig.6 is a three-dimensional view of the circumcision stapler of the present invention, with a part of a main bracket removed;
Fig.7 is a partially amplified sectional view of the circumcision stapler of the present invention;
Fig.8 is a structural view of the parallel array state of the staple cartridge of the circumcision stapler of the present invention;
Fig.9 is a structural view of an opening of the staple cartridge of the circumcision stapler of the present invention;
Fig.10 is a three-dimensional view of suturing staples and cushions of the circumcision stapler of the present invention;
Fig.11 is a structural and stressed view of the suturing staple and the cushion, in the fastened state, of the circumcision stapler of the present invention.

### DETAILED DESCRIPTION OF THE INVENTION

A specific embodiment of the present invention is described in detail in junction with the attached drawings.

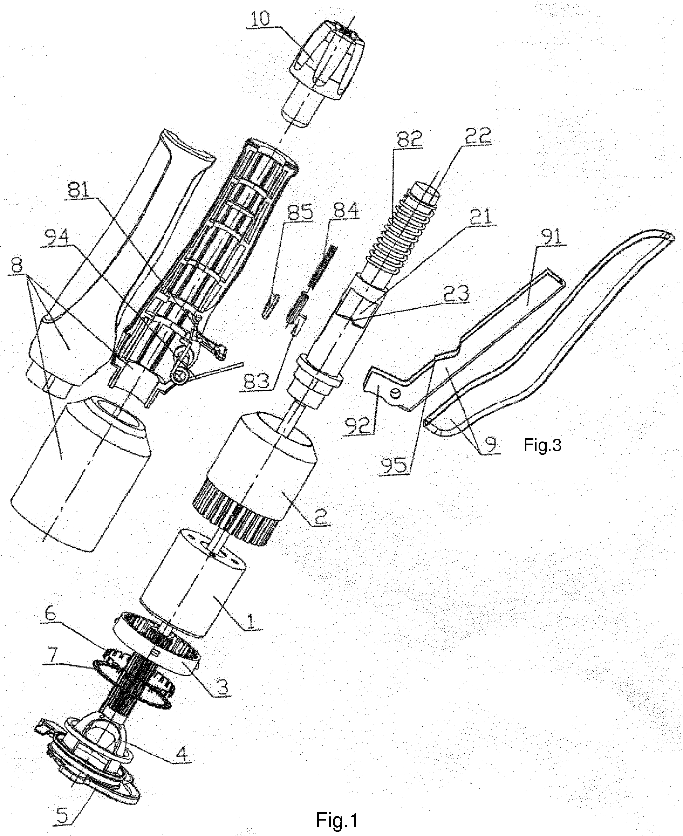

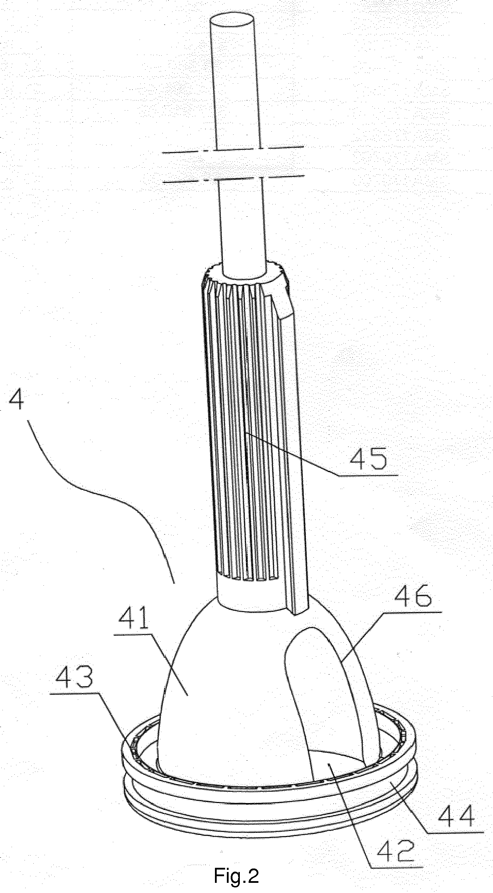

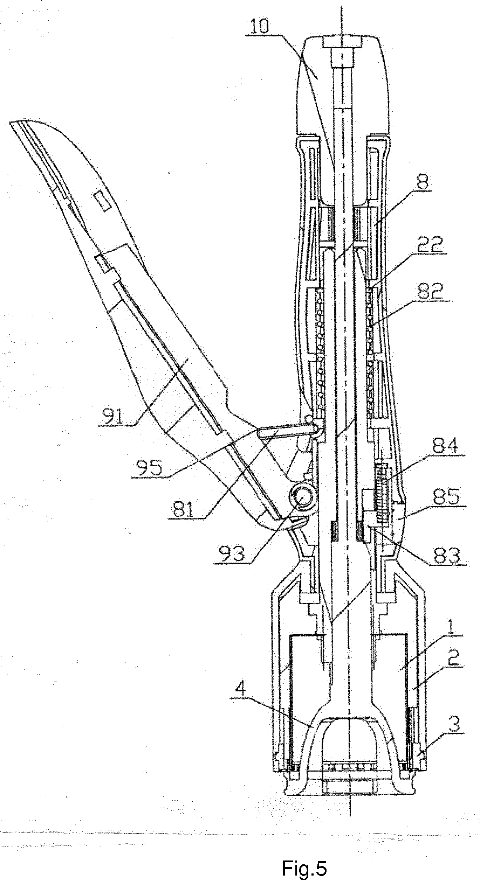

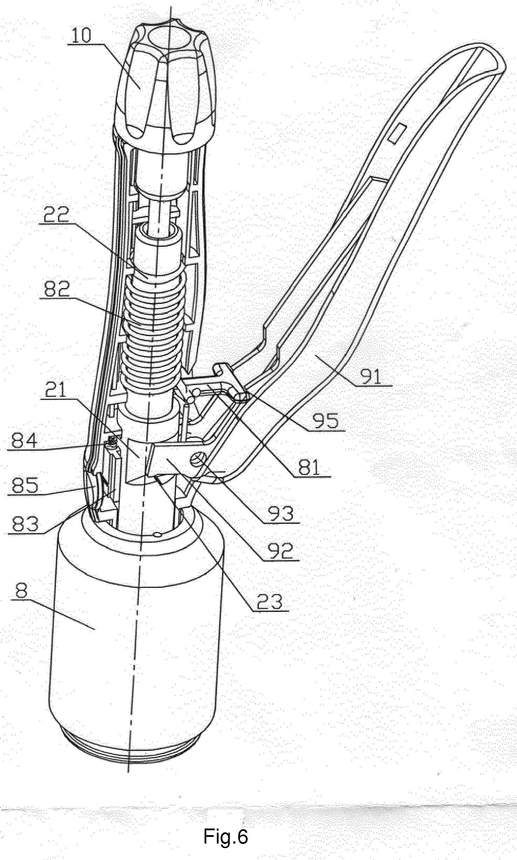

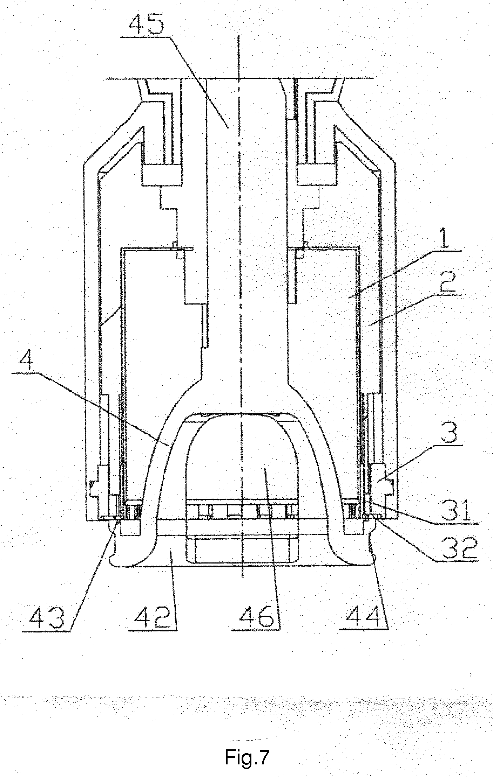

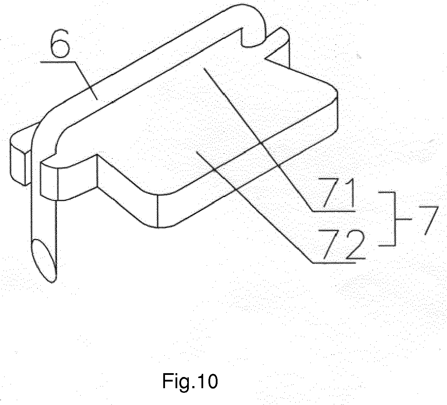

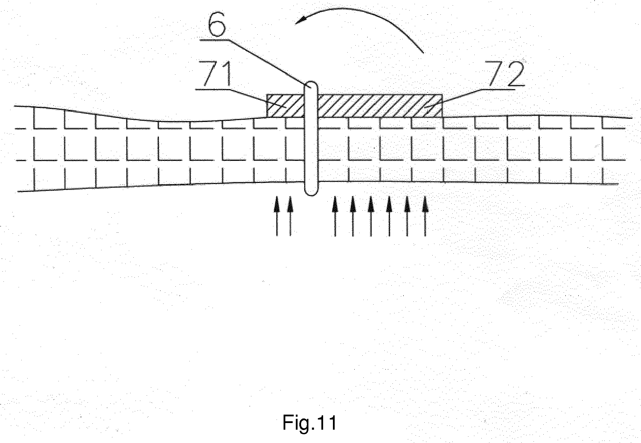

Figures 1-11 show the specific embodiment of the circumcision stapler of the present invention. The circumcision stapler includes an annular cutter 1, a staple pusher 2, a staple cartridge 3, a staple seat 4 and an external positioning member 5, wherein the staple seat 4 includes a staple seat body 41, a balanus accommodating cavity 42, staple grooves 43 and an internal positioning member 44, the staple seat body 41 has an inner cavity and an outer wall, the inner cavity has one end which is inward recessed and extends to form the balanus accommodating cavity 42, the outer wall is provided with a ring of staple grooves 43, the internal positioning member 44 is formed between the balanus accommodating cavity 42 and the outer wall of the staple grooves 43; wherein the annular cutter 1 is disposed in the staple pusher 2, the staple pusher 2, the staple cartridge 3 and the staple seat 4 are disposed in turn, the staple cartridge 3 is provided with a staple compartment 31, a cushion compartment 32 is disposed on one side of the staple compartment 31, the staple grooves 43 are disposed corresponding to the staple compartment 31 and the cushion compartment 32, the projection position of each one of suturing staples 6 in the staple compartment 31 is close to the inner side of the cushion compartment 32, the cushion compartment 32 contains single-sided staple cushions 7; wherein during use, the balanus is placed in the balanus accommodating cavity 42 and the prepuce is turned to the outer wall of the staple seat body 41 and hooped by the external positioning member 5 from the outside, the external positioning member 5 fixes the prepuce at the position of the internal positioning member 44, the staple cartridge 3 is aligned with the prepuce at the outer wall of the staple seat body 41, the annular cutter 1 and the staple pusher 2 are pushed toward the staple grooves 43, the annular cutter 1 cuts the prepuce, then the staple pusher 2 pushes the suturing staples 6 and a single-sided staple cushion 7 in the staple cartridge 31 to the staple grooves 43; wherein the suturing staples 6 are fastened on the prepuce, the suturing staples 6 are penetrated in one side of the single-sided staple cushion 7, one side of the single-sided staple cushion 7 with the suturing staples 6 is stably positioned, and the other side of the single-sided staple cushion 7 can flip along with the erection of the penis.

To use the device, the operator first puts the balanus into the balanus accommodating cavity 42 to protect the balanus and then turns the prepuce to the outer wall of the staple seat body 41. As shown in the figures, the staple seat body 41 can have a shape matched with the balanus. The operator adjusts the prepuce to determine the position of the internal positioning member 44, and after the position adjustment, hoops the prepuce using the external positioning member 5 from the outside to complete the primary positioning. The operator can hold the staple seat 4 with one hand and turn the prepuce with the other hand to completely expose the frenulum of prepuce and the inner plate structure, directly view the internal positioning member 44 and the frenulum position, position and adjust the frenulum of prepuce to prevent the frenulum of prepuce from entering the cutting and suturing zone of the staple grooves 43, and can rotate the staple seat 4 to fine tune the frenulum of prepuce to align with the middle line of the ventral side of the outer plate. When the sizes of the internal positioning member 44 and the external positioning member 5 are designed in a way that the internal positioning member 44 and the external positioning member 5 are assembled with each other in place and then firmly clamp the prepuce, the operator can loosen the external positioning member 5 to perform fine adjustment and then hoop and position the prepuce; when the internal positioning member 44 and the external positioning member 5 are designed to be assembled with each other in place while keeping a certain adjusting clearance, which means that the prepuce is not firmly clamped, the operator can pull the prepuce to fine adjust the position. Such two modes can both achieve accurate positioned cutting. After ensuring that the frenulum of prepuce is secure and well aligned and the inner and outer plates are adjusted in good conditions, the skins of the inner and outer plates of the prepuce are fixed and limited. A plurality of ejectors on the staple pusher 2 of the present invention are respectively inserted into the corresponding ones of the plurality of staple compartments on the staple cartridge 3. The ejectors can push the suturing staples 6 in the staple compartments 31 into the staple grooves 43; the suturing staples puncture the prepuce tissue, contact the staple holes 41 on the staple grooves 43, bend and mold the suturing staples; and the single-sided staple cushions 7 are driven together with the suturing staples 6 to be stapled on the prepuce tissue. During the staple pushing process, the annular cutter 1 moves along with the staple pusher 2 to cut the prepuce, while the staple cartridge 3 and the staple grooves 43 are matched on the upper and lower sides to press the prepuce. The single-sided staple cushions 7 and the suturing staples 6 are pressed on the outer side of the edge of the annular cutter 6. The annular cutter 1 can effectively reduce bleeding in the process of cutting the prepuce, having a good protection effect no matter during or after the surgery. By the straight-push stapling mode, the annular cutter 1 and the staple pusher 2 ensure stable cutting, and ensure that the single-sided cushions 7 and the suturing staples 6 can be stably pushed out and stapled. After the cutting and suturing is completed, remove the staple cartridge 3. Under the condition that the external positioning member 5 is not removed, observe the state of the suturing staple 6 at the cut. After ensuring that the all suturing staples 6 are firmly fastened, remove the external positioning member 5. If there is looseness, the external positioning member 5 can be removed after the corrective suturing is carried out, to avoid the risk of bleeding during surgery. By the above technical improvements on the internal positioning member 44 and the external positioning member 5, the surgery is safer,and more accurate, bleeding complications are reduced during the surgery, the operation quality and efficiency are enhanced, and the operation satisfaction degree of the patients is comprehensively improved. The staple compartments 31 and the cushion compartments 32 which are matched with each other are adopted to store the suturing staples 6 and the single-sided staple cushions 7. After the suturing staples 6 and the single-sided staple cushions 7 are accurately positioned, while the suturing staples 6 are respectively positioned on one side of each corresponding one of the single-sided staple cushions 7, the single-sided staple cushions 7 can keep good contact with the skin tissue after the penis erects, thus reducing the pressure between the suturing staples 6 and the skin tissue when the penis erects and improving the comfort level for the patients. Meanwhile, the pressure caused by the erection of the penis is undertaken by a single side of each one of the staple cushions 7, and the erecting penis generates an outward thrust to the suturing staples 6, so a leverage force for removing the suturing staples 6 toward the cutting edge is generated; the asymmetric cushions make the staple frame portion of the suturing staple closer to the cuttting edge, and then the suturing staples 6 more easily cut the cuttting edge and gets out of the cuttting edge by the poking effect of the leverage of the single-sided staple cushions 7, thus achieving the effect of quick staple removal, minimizing the cut scars after the surgery, making the cut natural and good-looking after healing, and generally improving the shaping effect at the cut.

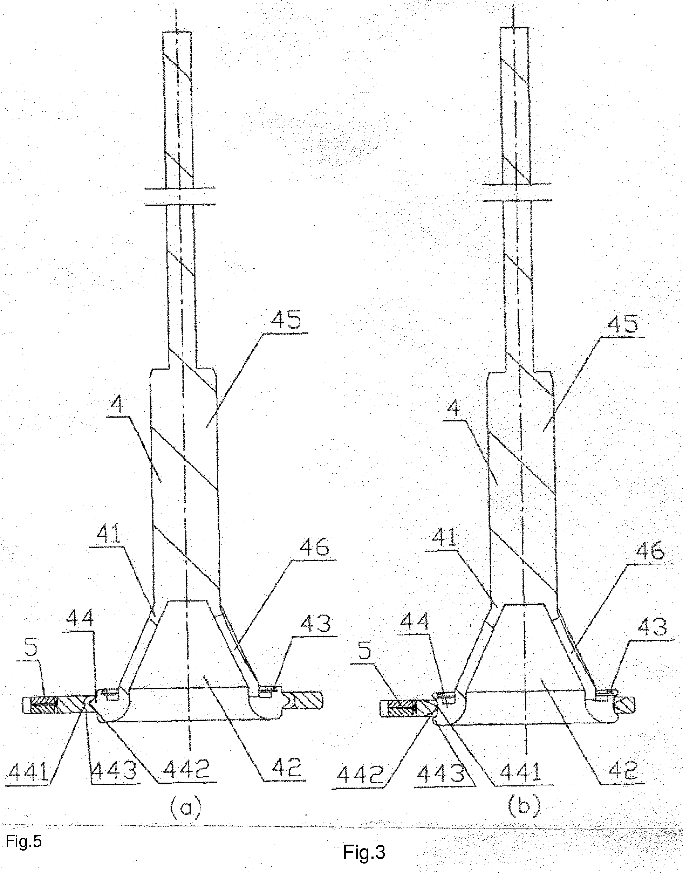

As an improved specific implementation mode, the opposite faces of the internal positioning member 44 and the external positioning member 5 are respectively provided with a clamping groove 441 and a clamping ridge 442 which are matched with each other; when the internal positioning member 44 and the external positioning member 5 are matched to perform positioning, the clamping ridge 442 is limited by the clamping groove 441 and groove walls 443 on two sides, and the prepuce is limited by a fold-like space formed by the clamping groove 441 and the clamping ridge 442. Fig.3(a) and Fig.3(b) show two modes of the arrangement of the clamping groove 441 and the clamping ridge 442. In the two arrangement modes, the clamping ridge 442 is always limited up and down by the groove walls 443; after positioning the prepuce, the external positioning member 5 is matched with the internal positioning member 44 to perform good limiting, capable of preventing the external positioning member 5 from separating the internal positioning member 44 during the prepuce adjustment and affecting the operation; and, the clamping ridge 442 and the groove walls 443 form fold-like clamping faces, increasing the area of contacting and rubbing with the prepuce, achieving a good effect of limiting the prepuce without causing crushing injury to the prepuce. Under the condition that the internal positioning member 44 and the external positioning member 5 hold the prepuce in a proper tight degree, the fold-like clearance formed by the clamping ridge 442 and the groove walls 443 can well limit the prepuce, and can also adjust the positions of the inner and outer plates of the prepuce relative to the internal positioning member 44 and the external positioning member 5 by a proper external force, thus achieving a dual effect of adjustment and positioning. As an optimized design, the clamping groove 441 and the clamping ridge 442 can both be designed into the annular shape to realize match, adjustment and positioning as a whole ring. Moreover, in order to improve the limiting effect, a plurality of groups of clamping grooves 441 and clamping ridges 442 respectively on the internal positioning member 44 and the external positioning member 5 can be arrayed to form a continuous wave-like structure.

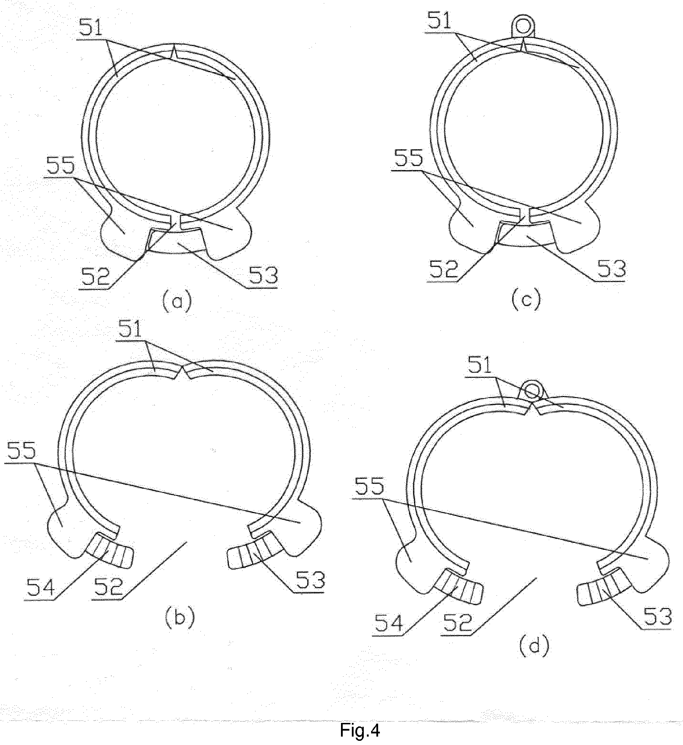

As an improved specific implementation mode, the external positioning member 5 is annular; the external positioning member 5 is a multi-segment or elastic one-segment positioning arc 51; the multi-segment positioning arc 51 is connected in turn and is able to open and close around the connection; the two outer ends of the positioning arc 51 are formed into openings 52; end portions of the positioning arc 51 at the openings 52 are respectively provided with an upper snap joint 53 and a lower snap joint 54 which are matched and snap-fitted, the snap-fitted multi-segment positioning arc 51 is ring formed; clutch handles 55 which are operated by people are disposed on the outer sides of the reverse faces of the upper snap joint 53 and the lower snap joint 54, and when two clutch handles 55 are operated by a reverse acting force, the upper snap joint 53 and the lower snap joint 54 are separated from each other. As shown in FIg.4, the external positioning member 5 can be set into a multi-segment positioning arc 51 upon demands. The figure shows a two-segment form. More segments can be designed upon demands. The multi-segment positioning arc 51 provides the external positioning member 5 with higher degree of freedom to open and close. The multi-segment positioning arc 51 can be made of elastic or undeformed materials upon demands. The one-segment positioning arc 51 is not shown in the figure. The one-segment positioning arc 51 is mainly made of elastic materials, opening through self elastic deformation, and enabling the end through the deformation to close and to be clamped by the upper snap joint 53 and the lower snap joint 54. Operators can select proper implementation modes upon own operation habits and the specific conditions of the patient to meet different needs. The specific operation of the clutch handles 55 is as follows. To implement the operation of fixing the prepuce, two clutch handles 55 are pressed toward each other such that the upper and lower snap joint are aligned and snap-fitted with each other to clamp the external positioning member 5. To remove the external positioning member 5 after the operation is completed, the operator presses the clutch handles 55 by applying acting forces which are opposite to the upper and lower directions of the plane of the external positioning member 5 to separate the upper snap joint 53 and the lower snap joint 54 of the external positioning member 5, and then dismantle the external positioning member 5. Such structure can be operated by a single hand, so the operation process is simplified. The above clutch handles 55 are disposed on the reverse outer sides of the upper snap joint 53 and the lower snap joint 54, not only at positions as shown in the figures, but also at positions away from the upper snap joint 53 and the lower snap joint 54, or at different positions relative to the upper snap joint 53 and the lower snap joint 54, as long as the clutch handles are disposed on the reverse sides and can be pressed by two mutually opposite acting forces to separate the upper snap joint 53 and the lower snap joint 54.

As an improved specific implementation mode, the upper snap joint 53 and the lower snap joint 54 are matched tooth-like structures; the tooth-like structures are uniformly arrayed in the circumferential direction; and the size of the space surrounded by the positioning arc 51 and the internal positioning member 44 is regulated by adjusting the number of the engaged teeth of the upper snap joint 53 and the lower snap joint 54. The operator operates the clutch handles 55 to engage a part of the teeth according to the prepuce thickness, achieving the effects of reliably limiting the prepuce to avoid displacement during operation and making a proper adjustment to the prepuce. After the prepuce position is correctly adjusted, the operator continuously pushes the clutch handles 55 such that the upper snap joint 53 and the lower snap joint 54 are completely engaged to fix the prepuce. Such mode makes the operation flexible and the adjustment convenient, allows the operator to accurately adjust and firmly fix the prepuce, and improves the operation quality and operation efficiency.

As an improved specific implementation mode, when the external positioning member 5 hoops the prepuce from the outside at the position of the internal positioning member 44, the clearance between the internal positioning member 44 and the external positioning member 5 enables the positioned prepuce to adjust in position. Due to a proper clearance between the internal positioning member 44 and the external positioning member 5, the fixed prepuce can be displaced relative to the internal positioning member 44 or the external positioning member 5 by a proper force. Such implementation mode is preferably employed to help the operator to freely adjust the prepuce to be cut without releasing the fixation between the internal positioning member 44 and the external positioning member 5, so the surgery is more convenient and efficient.

As an improved specific implementation mode, the staple seat 4 also includes a multi-ridge columnar connecting bridge 45 which is connected to the other end of the staple seat 41 opposite to the balanus accommodating cavity 42, the internal positioning member 44 is disposed on the outer circumference of the opening end o the balanus accommodating cavity 42 on the staple seat 41; and the staple seat 41is formed with at least one view window 46 which runs through the balanus accommodating cavity 42 and through which people can observe the positions of the balanus and the prepuce. The multi-ridge columnar connecting bridge 45, serving as an extending structure that is integrated with the staple seat 41, is used to be matched with the staple cartridge 3 of the prepuce cutting and suturing device to perform positioning, is beneficial to accurate and quick assembling of the staple cartridge 3 and the staple seat 4 of the prepuce cutting and suturing device. The accurate positioning of the the staple cartridge 3 and the staple seat 4 helps to ensure the prepuce cutting and suturing effect and improves the operation quality. The internal positioning member 44 disposed on the outer circumference of the opening end of the balanus accommodating cavity 42 on the staple seat body 41 plays the roles of determining the secure cutting position of the inner plate, positioning the frenulum at a place away from the cutting and suturing area, and cooperating with the external positioning member 5 to fix and adjust the prepuce. In order to further improve the positioning accuracy and the direct observation of the frenulum of prepuce, a view window 46 through which the balanus in the balanus accommodating cavity 42 is provided. After turning the prepuce, the operator can see the positions of the balanus in the balanus accommodating cavity 42 and the connected frenulum, and more directly observe the situations of the entire frenulum on the outer and inner sides of the internal positioning member 44, so the positioning is more accurate, the adjustment is more convenient, injuries of the frenulum are avoided, and the operation safety is improved.

As an improved specific implementation mode, a main bracket 8 and a handle 9 are also included, wherein the main bracket 8 is internally fixedly provided with the staple cartridge 3, the main bracket 8 is internally movably provided with the annular cutter 1 and the staple pusher 2, the handle 9 is rotationally disposed on the main bracket 8 and penetrated in the main bracket 8 to match with the staple pusher 2 in a linked way; the handle 9 includes an outer rotating arm 91 and inner pushing arms 92, the outer rotating arm 91 and the inner pushing arms 92 are rotationally disposed on the main bracket 8 through a middle rotating shaft 93, the rotating shaft 93 is provided with a torsional spring 94, the torsional spring 94 is matched with the outer rotating arm 91 and the main bracket 8, two inner pushing arms 92 are provided, the two inner pushing arms 92 extend into the symmetrical pushing grooves 21on the staple pusher 2; the main bracket 8 is internally provided with a reset spring 82, the reset spring 82 is matched with a stopper 22 disposed on the staple pusher 2, the stopper 22 compresses the reset spring 82 when the staple pusher 2 moves forward, the reset spring 82 drives the staple pusher 2 to reset when the force applied to the staple pusher 2 is removed; a projection 23 is disposed at the bottom of each one of the pushing grooves 21, and when the inner pushing arms 92 are pressed against the bottoms of the pushing grooves 21 and rotationally pass through the projections 23, the staple pusher 2 is sprung back by the force of the reset spring 82 and gives the prompt that the staple is pushed in place.

In use, a doctor holds the main bracket 8 with a hand, controls the staple seat 4 and the main bracket 8 to clamp the prepuce of the patient. Specifically, the staple grooves 43 on the staple seat 4 and the staple cartridge 3 on the main bracket 8 clamp the prepuce from the upper and lower sides. The doctor presses the handle 9 to control the annular cutter 1 and the staple pusher 2 to move to realize straight-push cutting of the prepuce. The annular cutter 1, the staple pusher 2 and the staple cartridge 3 can be well positioned and limited in the main bracket 8 and stably matched with one another to facilitate assembling and match between the structure in the main bracket 8 and the structure on a balanus cover 5. The handle 9 has a simple structure, but relatively high flexibility, and together with the rotating shaft 93 forms a lever structure through the outer rotating arm 91 and the inner pushing arms 92. The doctor can press the outer rotating arm 91 to drive the inner pushing arms 92 to downward push the staple pusher 2, thus realizing staple pushing and cutting. To realize resetting, the torsional spring 94 and the reset spring 82 work together to realize stable resetting. The elastic force of the torsional spring 94 drives the handle 9 to reset, while the elastic force of the reset force 82 drives the staple pusher 2 to reset, so the handle 9 and the staple pusher 2 reset in good conditions without clamping and are convenient to use. The two inner pushing arms 92 are provided and are matched with the symmetric pushing grooves 21 to help smooth pushing of the staple pusher 2, ensuring the smooth movement of the staple pusher 2, consistent and accurate staple pushing and cutting. During practical operation, if the operator presses the handle 9 inadequately or excessively, it is likely that the suturing staples 6 are missing or the suturing staples 6 are deformed by excessive tension, affecting the operation quality. Therefore, the pushing grooves 21 are provided with projections 23. The specific positions of the projections 23 in the pushing grooves 21 correspond to proper pushing positions of the suturing staples 6. When the operator presses the handle 9, the inner pushing arms 92 are pressed against the bottoms of the pushing grooves 21 and rotationally move forward to push the staple pusher 2. When the suturing staples 6 are pushed to the proper working positions, which means that the suturing staples are already bent and staple the prepuce without excessive bending, the inner pushing arms 92 exactly arrive at the projections 23. When the inner pushing arms 92 pass through the projections 23, the operator has a disengaging hand feel; the staple pusher 2 springs back by the force of the reset spring 82, the operator can feel that the staple is pushed in place, thus ensuring the operation quality. The suturing staples 6 can staple the wound in good conditions. The projections 23 can be smooth projections disposed at the bottoms of the pushing grooves 21, and do not affect the thrust applied by the operator to push the staple pusher 2, and can also be any projecting structures that can conduct the above functions.

As an improved specific implementation mode, the main bracket 8 is also provided with a pressing member 83 and a pressing spring 84; the pressing member 83 and the pressing spring 84 are matched and are installed on the main bracket 8; when the staple seat 4 is installed in the main bracket 8, the staple seat 4 is pressed against the pressing member 83 to compress the pressing spring 84; the far end of the staple seat 4 penetrates out of the main bracket 8 and is detachably provided with a locking knob 10; when rotating the locking knob 10, a person determines the position of the pressing member 83 via a locking mark window 85 disposed on the main bracket 8; the main bracket 8 is also provided with a rotary clamp 81, the outer rotating arm 91 is provided with clamping points 95, and when the rotary clamp 81 is pressed against the clamping points 95, the outer rotating arm 91 is limited. The existing suturing device does not have the function of prompting that the staple seat 4 is installed in place, and the prepuce cannot be seen after the staple seat 4 and the main bracket 8 are installed. The operators all rotate the locking knob 10 at the staple seat 4 to the largest extent to complete fixation of the staple seat 4 and the main bracket 8. However, the case that the staple seat 8 and the main bracket 8 are not firmly fixed because the locking knob is not rotated to the end or the case that prepuce is compressed because the locking knob is excessively screwed still exists, and such two cases both have defects of non-uniform journey, which results in that the suturing staples 6 are not bent well or the suturing staples 6 are excessively pressed to affect the suturing quality in the process of pressing the handle 9 . Therefore, a structure including the pressing member 83 and the pressing spring 84 is provided; the pressing member 83 is pressed against and in the main bracket 8 by means of the pressing spring 84; when the main bracket 8 is installed from one side of the staple seat 4, the staple seat 4 shoulders the pressing member 83 off, and the pressing member 83 is limited by the staple seat 4 and the pressing spring 84. By setting the locking mark window 85, the position of the pressing member 83 can be directly observed and determined. The proper installation position can be set in advance, and when the locking knob 10 locks the staple seat 4 until the pressing member 83 moves to the pre-set proper installation position, the fixation of the staple seat 4 and the main bracket 8 is completed. In this way, it is ensured that the annular cutter 1 and the staple pusher 2 have the most appropriate journeys and can cut the prepuce and bend the suturing staples well, the operation quality is improved, and missing stapling of the suturing staples 6 or deformation of the staples because of excessive compression is avoided. In order to prevent the doctor from pushing the staple pusher 2 by mistake before the positioning of the balanus and the prepuce is completed, the rotary clamp 81 and the clamping points 95 are provided. The outer rotating arm 91 cannot rotate inward to press the prepuce after being pressed against by the rotary clamp 81, ensuring the safety of the prepuce operation and lowering the probability of occurrence of accidents. On the other side, the suturing device is conveniently stored. Under the storage condition that the suturing device is internally provided with the suturing staples 6 and the single-sided staple cushions 7, the mis-operation of pushing the suturing taples and the single-sided staple cushions 7 is prevented, and convenient is brought to management of the suturing device and the normal use of the suturing device in subsequent operations.

As an improved specific implementation mode, the staple grooves 43 are arrayed in parallel in the circumferential direction; intervals between adjacent staple grooves 43 are all smaller than or equal to 1.0mm, and are all greater than or equal to 0.4mm; and the staple compartment 31 and the cushion compartment 32 are disposed in a way of corresponding to the staple grooves 43. The staple grooves 43 are orderly arrayed in a ring shape; the staple grooves 43 are sequentially arrayed in an end-to-end way in parallel. Due to the denser and orderly array structure, the staple grooves 43 are arranged in orderly intervals and better seal the clearances, which helps the whole ring of cut to heal and obtain an orderly and good-looking appearance. Stable stapling effects are achieved at all positions on the circumference. Compared with the existing structure, in this embodiment, the intervals between the staple grooves 43 are set to be smaller, and the staple compartments 31 and the cushion compartments 32 are correspondingly disposed. In this way, the blood vessels can be better sealed, the probability of missing stapling of the blood vessels when the prepuce cutting and suturing device is used to cut the prepuce is reduced, hemostasis is more reliable, and the probability of post-operative bleeding is smaller. Blood vessels on the prepuce are not uniformly distributed. More blood vessels are distributed at some positions, for example the back side and the ventral side of the cross section of the penis. In order to further improve the effect of sealing the blood vessels, upon clinical experience, blood vessels with a size of smaller than or equal to 1.0mm can be pressed to stop bleeding after the operation, and it is difficult to stop the bleeding of the blood vessels with a size of greater than or equal to 1.0mm by pressing, and the blood vessels tend to retract and cause hematoma after the operation. Relative to the existing relatively large spaced arrangement structure, the intervals between adjacent staple grooves 43 are set to be below 1.0mm. A tester can select different interval size to do experiments, for example several standard interval sizes, 1.0mm, 0.9mm, 0.8mm, 0.7mm, 0.6mm, 0.5mm, 0.4mm, etc. The arrangement structure of the present invention can greatly lower the probability of staple missing on the blood vessels with diameters of greater than 1.0mm on the prepuce, achieve a good effect of sealing the blood vessels with the suturing staples when the prepuce cutting and suturing device is used to cut the prepuce, improve the hemostasis reliability during and after the operation, reduce the occurrence of post-operative hematoma, and obtain a better sealing effect. According to clinical experiments, intervals below 0.4mm can cause decline in the tensile elasticity of the tissue between the staples, and tend to form narrow ring after the penis erects, wherein the narrow ring hooping the penis can cause prepuce edema. Intervals which are over or equal to 0.4mm can overcome the above defects and can ensure blood supply at the cut, quick cut healing, low cracking probability, and small post-operative scars.

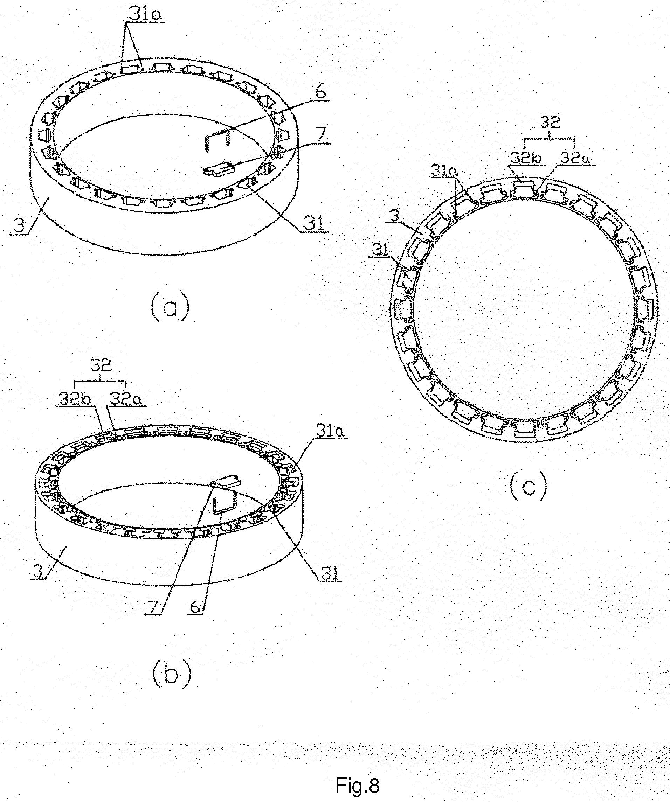

As an improved specific implementation mode, each one of the staple compartments 31 has limiting grooves 31a on one two sides for positioning suturing staple feet, the projection positions of the limiting grooves 31a are positioned at the inner side of each one of the cushion compartments 32, the limiting grooves 31a in each one of the staple compartments 31 a limit the suturing staples 6; each one of the cushion compartments 32 includes a staple loading cushion cavity side 32a and a staple levering cushion cavity side 32b, the staple loading cushion cavity side 32a and the limiting grooves 31a are correspondingly disposed in terms of the projection positions, the staple levering cushion cavity side 32b is positioned on the outer side of the staple loading cushion cavity side 32a; the single-sided staple cushion 7 includes a staple loading portion 71 and a staple levering portion 72 which are integrated, the staple loading cushion cavity side 32a and the staple levering cushion cavity side 32b respectively accommodate the staple loading portion 71 and the staple levering portion 72, the staple loading portion 71 is penetrated with the suturing staples 6, the staple levering portion 72 is a turnable side; after the suturing staple 6 is fastened on the prepuce, the staple levering portion 72 faces the root of the penis; and when the penis erects, the staple levering portion 72 is stressed to turn toward the cutting direction. By setting the limiting grooves 31 a, the staple compartments 31 are adapted to the suturing staples 6, and the feet on two sides of the suturing staples are limited in the limiting grooves 31a to ensure that the suturing staples 6 are stably and accurately pushed toward the staple grooves 41. The projections of the limiting grooves 31 a are positioned on the inner sides of the cushion compartments 32, forming a structure in which the suturing staples 6 deviate to one side, and when pushed toward the lower single-sided staple cushions 7, the suturing staples 6 penetrate through the single side and close to the inner side, which means that the suturing staples 6 close to the cut after being fastened. The pushing blocks on the staple pusher 2 are adapted to the shapes of the staple compartments 31, while the staple compartments 31 are adapted to the lower single-sided staple cushions 7. Therefore, the pushing blocks on the staple pusher 2 can stably press the single-sided staple cushions 7 when pushing the staples in place such that the single-sided staple cushions 7 are accurately limited. In this way, the single-sided staple cushions 7 and the suturing staples 6 can be smoothly matched and stapled on the prepuce tissue, ensuring the hemostasis effect and ensuring the comfort level of contact between the single-sided staple cushions 7 and the skin. As shown in Figures 8-11, the staple loading portion 71 on each one of the single-sided staple cushions 7 is installed on the staple loading cushion cavity side 32a, while the staple levering portion 72 is installed on the staple levering cushion cavity side 32b. The structure is stable and conveniently positioned, pushes the single-sided staple cushions 7 toward the area where the limiting grooves 31 a are positioned, and is beneficial to pushing and bending of the suturing staples 6. The suturing staples 6 together with the staple loading portions 71 of the single-sided staple cushions 7 are well stapled and fixed on the skin, achieving a good hemostasis effect, ensuring the staple levering portions 72 on the other side have certain flexibility, and facilitating levering and turning of the suturing staples when the penis erects later. When the penis erects, the staple levering portions 72 can turn along with the penis surface, thus better supporting the penis. The staple levering portions 72 can turn along with the thrust force caused by the expansion of the penis. The staple levering portions 72 turns, like a lever, to lever and pull the suturing staples 6, so the suturing staples 6 more easily cut the cut edge and get out of the cut. The staple levering portions 72 help staple removal.

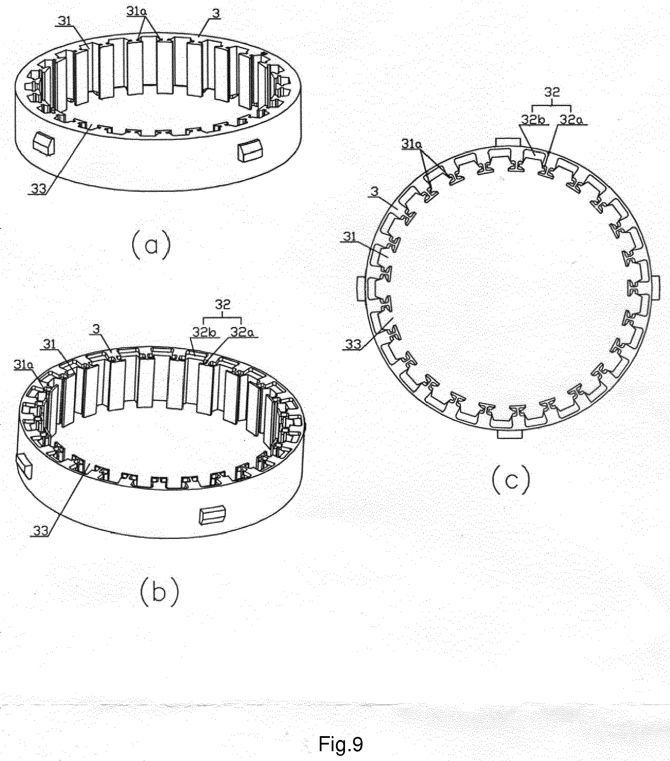

As an improvement, the staple compartments 31 and the cushion compartments 32 are disposed close to the inner wall of the staple cartridge 3, and form axial run-through openings 33 on the inner wall of the staple cartridge 3. In the process of installing the staple compartments 31 and the cushion compartments 32 close to the inner wall of the staple cartridge 3, the excessively thin inner wall of the staple cartridge 3 are easily damaged and result in processing difficulties. Therefore, by employing the above implementation mode, as shown in Fig.9, the inner wall of the staple cartridge 3 is directly removed, which means that the openings 33 are configured to make the staple compartments 31 and the cushion compartments 32 further close to the edge of the staple cartridge 3. By adopting the leverage principle, the suturing staples 6 more easily get out from the cut edge. The openings 33 are only required to be smaller than the relative widths of the staple compartments 31 and the cushion compartments 32, or the widths of shapes of the single-sided staple cushions 7 are greater than the widths of the openings 33, thus avoiding affecting normal storage and push of the suturing staples 6 and the single-sided staple cushions 7 respectively in the staple compartments 31 and the cushion compartments 32.

## Claims

1. Circumcision stapler, **characterized by** comprising an annular cutter (1), a staple pusher (2), a staple cartridge (3), a staple seat (4) and an external positioning member (5), wherein the staple seat (4) comprises a staple seat body (41), a balanus accommodating cavity (42), staple grooves (43) and an internal positioning member (44), the staple seat body (41) has an inner cavity and an outer wall, the inner cavity has one end which is inward recessed and extends to form the balanus accommodating cavity (42), the outer wall is provided with a ring of staple grooves (43), the internal positioning member (44) is formed between the balanus accommodating cavity (42) and the outer wall of the staple grooves (43);
wherein the annular cutter (1) is disposed on inner side of the staple pusher (2), the staple pusher (2), the staple cartridge (3) and the staple seat (4) are disposed in turn, the staple cartridge (3) is provided with staple compartments (31), a cushion compartment (32) is disposed on one side of each one of the staple compartments (31), the staple grooves (43) are disposed corresponding to the staple compartments (31) and the cushion compartments (32), the projection position of each one of suturing staples (6) in each one of the staple compartments (31) is close, namely 0,1mm to 2,0 mm, in particular 0,5 to 1,0 mm, to the inner side of each corresponding one of the cushion compartments (32), the cushion compartments (32) contain single-sided staple cushions (7);

2. Circumcision stapler according to claim 1, **characterized in that** the Circumcision stapler is adapted in such a manner that during use, the balanus is placable in the balanus accommodating cavity (42) and the prepuce is turnable to the outer wall of the staple seat body (41) and hooped by the external positioning member (5) from the outside, the external positioning member (5) can fix the prepuce at the position of the internal positioning member (44), the staple cartridge (3) can aligne with the prepuce at the outer wall of the staple seat body (41), the annular cutter (1) and the staple pusher (2) are pushable toward the staple grooves (43), the annular cutter (1) can cut the prepuce, then the staple pusher (2) can push the suturing staples (6) and single-sided staple cushions (7) in the staple cartridge (31) to the staple grooves (43); wherein the suturing staples (6) are fastened on the prepuce, the suturing staples (6) are respectively penetrated in one side of each corresponding one of the single-sided staple cushions (7), wherein one side of each one of the single-sided staple cushions (7) with the suturing staple (6) is stably positioned, and the other side of the single-sided staple cushion (7) can flip along with the erection of the penis.

3. Circumcision stapler according to at least one of the claims 1 and 2, **characterized in that** the opposite faces of the internal positioning member (44) and the external positioning member (5) are respectively provided with a clamping groove (441) and a clamping ridge (442) which are matched with each other; when the internal positioning member (44) and the external positioning member (5) are matched to perform positioning, the clamping ridge (442) is limited by the clamping groove (441) and groove walls (443) on two sides, and the prepuce is limited by a fold-like space formed by the clamping groove (441) and the clamping ridge (442).

4. Circumcision stapler according to at least one of the preceding claims, **characterized in that** the external positioning member (5) is annular; the external positioning member (5) is a multi-segment or elastic one-segment positioning arc (51); the multi-segment positioning arc (51) is connected in turn and is able to open and close around the connection; the two outer ends of the positioning arc (51) are formed into openings (52); end portions of the positioning arc (51) at the openings (52) are respectively provided with an upper snap joint (53) and a lower snap joint (54) which are matched and snap-fitted, the snap-fitted multi-segment positioning arc (51) is ring formed; clutch handles (55) are disposed on the outer sides of the reverse faces of the upper snap joint (53) and the lower snap joint (54), such that when two clutch handles (55) are operated by a reverse acting force, the upper snap joint (53) and the lower snap joint (54) are separated from each other.

5. Circumcision stapler according to at least one of the preceding claims, **characterized in that** the circumcision stapler is adapted in such a manner that when the external positioning member (5) hoops the prepuce from the outside at the position of the internal positioning member (44), the clearance between the internal positioning member (44) and the external positioning member (5) enables the positioned prepuce to adjust in position.

6. Circumcision stapler according to at least one of the preceding claims, **characterized in that** the staple seat (4) also comprises a multi-ridge columnar connecting bridge (45) which is connected to the other end of the staple seat (41) opposite to the balanus accommodating cavity (42), the internal positioning member (44) is disposed on the outer circumference of the opening end of the balanus accommodating cavity (42) on the staple seat (41); and the staple seat (41) is formed with at least one view window (46) which runs through the balanus accommodating cavity (42) and through which operator can observe the positions of the balanus and the prepuce.

7. Circumcision stapler according to at least one of the preceding claims, **characterized by** also comprising a main bracket (8) and a handle (9), wherein the main bracket (8) is internally fixedly provided with the staple cartridge (3), the main bracket (8) is internally movably provided with the annular cutter (1) and the staple pusher (2), the handle (9) is rotationally disposed on the main bracket (8) and penetrated in the main bracket (8) to match with the staple pusher (2) in a linked way;

8. Circumcision stapler according to at claim 7, **characterized in that** the handle (9) comprises an outer rotating arm (91) and inner pushing arms (92), the outer rotating arm (91) and the inner pushing arms (92) are rotationally disposed on the main bracket (8) through a middle rotating shaft (93), the rotating shaft (93) is provided with a torsional spring (94), the torsional spring (94) is matched with the outer rotating arm (91) and the main bracket (8), two inner pushing arms (92) are provided, the two inner pushing arms (92) extend into the symmetrical pushing grooves (21) on the staple pusher (2); the main bracket (8) is internally provided with a reset spring (82), the reset spring (82) is matched with a stopper (22) disposed on the staple pusher (2), the stopper (22) compresses the reset spring (82) when the staple pusher (2) moves forward, the reset spring (82) drives the staple pusher (2) to reset when the force applied to the staple pusher (2) is removed; a projection (23) is disposed at the bottom of each one of the pushing grooves (21), such that when the inner pushing arms (92) are pressed against the bottoms of the pushing grooves (21) and rotationally pass through the projections (23), the staple pusher (2) is sprung back by the force of the reset spring (82) and gives the prompt that the staple is pushed in place.

9. Circumcision stapler according to at least one of the claims 7 and 8, **characterized in that** the main bracket (8) is also provided with a pressing member (83) and a pressing spring (84); the pressing member (83) and the pressing spring (84) are matched and are installed on the main bracket (8);

10. Circumcision stapler according to claim 9, **characterized in that** the circumcision stapler is adapted in such a manner that when the staple seat (4) is installed in the main bracket (8), the staple seat (4) is pressed against the pressing member (83) to compress the pressing spring (84); the far end of the staple seat (4) penetrates out of the main bracket (8) and is detachably provided with a locking knob (10).

11. Circumcision stapler according to claim 10, **characterized in that** the circumcision stapler is adapted in such a manner that when rotating the locking knob (10), the position of the pressing member (83) is determinable via a locking mark window (85) disposed on the main bracket (8).

12. Circumcision stapler according to one of the claims 10 and 11, **characterized in that** the main bracket (8) is also provided with a rotary clamp (81), the outer rotating arm (91) is provided with clamping points (95), such when the rotary clamp (81) is pressed against the clamping points (95), the outer rotating arm (91) is limited.

13. Circumcision stapler according to at least one of the preceding claims, **characterized in that** the staple grooves (43) are arrayed in parallel in the circumferential direction; intervals between adjacent staple grooves (43) are all smaller than or equal to 1.0mm, and are all greater than or equal to 0.4mm; and the staple compartments (31) and the cushion compartments (32) are disposed in a way of corresponding to the staple grooves (43).

14. Circumcision stapler according to at least one of the preceding claims, **characterized in that** each one of the staple compartments (31) has limiting grooves (31a) on one two sides for positioning suturing staple feet, the projection positions of the limiting grooves (31a) are positioned at the inner side of each one of the cushion compartments (32), the limiting grooves (31a) in the staple compartments (31) limit the position of the suturing staples (6); each one of the cushion compartments (32) comprises a staple loading cushion cavity side (32a) and a staple levering cushion cavity side (32b), the staple loading cushion cavity side (32a) and the limiting grooves (31a) are correspondingly disposed in terms of the projection positions, the staple levering cushion cavity side (32b) is positioned on the outer side of the staple loading cushion cavity side (32a); each one of the single-sided staple cushions (7) comprises a staple loading portion (71) and a staple levering portion (72) which are integrated, the staple loading cushion cavity side (32a) and the staple levering cushion cavity side (32b) respectively accommodate the staple loading portion (71) and the staple levering portion (72), the staple loading portion (71) is penetrated or penetratable with the suturing staples (6), the staple levering portion (72) is a turnable side such that after the suturing staple (6) is fastened on the prepuce, the staple levering portion (72) faces the root of the penis; and when the penis erects, the staple levering portion (72) is stressed to turn toward the cutting direction.

15. Circumcision stapleraccording to at least one of the preceding claims, **characterized in that** the staple compartments (31) and the cushion compartments (32) are disposed close, namely 0,1mm to 2,0 mm, in particular 0,5 to 1,0 mm, to the inner wall of the staple cartridge (3), and form axial run-through openings (33) on the inner wall of the staple cartridge (3).
